# EUROPÄISCHE PATENTSCHRIFT

(11) **EP 0 862 633 B1**
(45) Veröffentlichungstag und Bekanntmachung des Hinweises auf die Patenterteilung: **02.02.2005**
(21) Anmeldenummer: 96941013.3
(22) Anmeldetag: 22.11.1996
(51) Int. Cl.: C12N 15/37, C07K 14/025, C07K 14/16, G01N 33/569, A61K 39/12, A61K 48/00

(54) **VP-ANTIGENE DES JC-VIRUS**
VIRUS PROTEIN ANTIGENS OF THE JC VIRUS
ANTIGENES VP DU VIRUS JC

(30) Priorität: 22.11.1995 DE 19543553
(43) Veröffentlichungstag der Anmeldung: 09.09.1998
(73) Patentinhaber: DEUTSCHES PRIMATENZENTRUM GESELLSCHAFT MBH, 37077 Göttingen (DE)
(72) Erfinder: LÜKE, Wolfgang, D-37077 Göttingen (DE); HUNSMANN, Gerhard, D-37077 Göttingen (DE); WEBER, Thomas, D-22087 Hamburg (DE)
(74) Vertreter: Weiss, Wolfgang, Dipl.-Chem. Dr.
(86) Internationale Anmeldenummer: PCT/EP1996/005177
(87) Internationale Veröffentlichungsnummer: WO 1997/019174

(56) Entgegenhaltungen:
- WO-A-94/20137
- PROCEEDINGS OF THE NATIONAL ACADEMY OF SCIENCES OF USA, Bd. 90, Nr. 11, 1.Juni 1993, WASHINGTON US, Seiten 5062-5065, XP002027809 T. IIDA ET AL.: "Origin of JC polyomavirus variants associated with progressive multifocal leukoencephalopathy"
- AIDS (U.S.), Bd. 8, Nr. 1, Januar 1994, Seiten 49-57, XP000647605 T. WEBER ET AL.: "Progressive multifocal leukoencephalopathy diagnosed by amplification of JC virus-specific DNA from cerebrospinal fluid"

### SEQUENZPROTOKOLL

(1) ALLGEMEINE ANGABEN:
   (i) ANMELDER:
      (A) NAME: Deutsches Primatenzentrum GmbH
      (B) STRASSE: Kellnerweg 4
      (C) ORT: Goettingen
      (E) LAND: Deutschland
      (F) POSTLEITZAHL: 37077
   (ii) BEZEICHNUNG DER ERFINDUNG: VP-Antigene des JC-Virus
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.30 (EPA)
(2) ANGABEN ZU SEQ ID NO: 1:
   (i) SEQUENZKENNZEICHEN:
      (A) LÄNGE: 1121 Basenpaare
      (B) ART: Nucleotid
      (C) STRANGFORM: beides
      (D) TOPOLOGIE: linear
   (ix) MERKMAL:
      (A) NAME/SCHLÜSSEL: CDS
      (B) LAGE:39..1100
   (xi) SEQUENZBESCHREIBUNG: SEQ ID NO: 1:
   GTACGGGACT GCAGCACCTG CTCTTGAAGC ATATGAAG ATG GCC CCA ACA AAA        53
                                             Met Ala Pro Thr Lys
                                               1               5
   AGA AAA GGA GAA AGG AAG GAC CCC GTG CAA GTT CCA AAA CTT CTT ATA     101
   Arg Lys Gly Glu Arg Lys Asp Pro Val Gln Val Pro Lys Leu Leu Ile
                    10                  15                  20
   AGA GGA GGA GTA GAA GTT CTA GAA GTT AAA ACT GGG GTT GAC TCA ATT     149
   Arg Gly Gly Val Glu Val Leu Glu Val Lys Thr Gly Val Asp Ser Ile
                25                  30                  35
   ACA GAG GTA GAA TGC TTT TTA ACT CCA GAA ATG GGT GAC CCA GAT GAG     197
   Thr Glu Val Glu Cys Phe Leu Thr Pro Glu Met Gly Asp Pro Asp Glu
            40                  45                  50
   CAT CTT AGG GGT TTT AGT AAG TCA ATA TCT ATA TCA GAT ACA TTT GAA        245
   His Leu Arg Gly Phe Ser Lys Ser Ile Ser Ile Ser Asp Thr Phe Glu
        55                  60                  65
   AGT GAC TCC CCA AAT AGG GAC ATG CTT CCT TGT TAC AGT GTG GCC AGA        293
   Ser Asp Ser Pro Asn Arg Asp Met Leu Pro Cys Tyr Ser Val Ala Arg
    70                  75                  80                  85
   ATT CCA CTA CCC AAT CTA AAT GAG GAT CTA ACC TGT GGA AAT ATA CTC        341
   Ile Pro Leu Pro Asn Leu Asn Glu Asp Leu Thr Cys Gly Asn Ile Leu
                    90                  95                 100
   ATG TGG GAG GCT GTG ACC TTA AAA ACT GAG GTT ATA GGG GTG ACA AGT        389
   Met Trp Glu Ala Val Thr Leu Lys Thr Glu Val Ile Gly Val Thr Ser
               105                 110                 115
   TTG ATG AAT GTG CAC TCT AAT GGG CAA GCA ACT CAT GAC AAT GGT GCA        437
   Leu Met Asn Val His Ser Asn Gly Gln Ala Thr His Asp Asn Gly Ala
           120                 125                 130
   GGG AAG CCA GTG CAG GGC ACC AGC TTT CAT TTT TTT TCT GTT GGG GGG        485
   Gly Lys Pro Val Gln Gly Thr Ser Phe His Phe Phe Ser Val Gly Gly
       135                 140                 145
   GAG GCT TTA GAA TTA CAG GGG GTG CTT TTT AAT TAC AGA ACA AAG TAC        533
   Glu Ala Leu Glu Leu Gln Gly Val Leu Phe Asn Tyr Arg Thr Lys Tyr
   150                 155                 160                 165
   CCA GAT GGA ACA ATT TTT CCA AAG AAT GCC ACA GTG CAA TCT CAA GTC        581
   Pro Asp Gly Thr Ile Phe Pro Lys Asn Ala Thr Val Gln Ser Gln Val
                   170                 175                 180
   ATG AAC ACA GAG CAC AAG GCG TAC CTA GAT AAG AAC AAA GCA TAT CCT        629
   Met Asn Thr Glu His Lys Ala Tyr Leu Asp Lys Asn Lys Ala Tyr Pro
               185                 190                 195
   GTT GAA TGT TGG GTT CCT GAT CCC ACC AGA AAT GAA AAC ACA AGA TAT        677
   Val Glu Cys Trp Val Pro Asp Pro Thr Arg Asn Glu Asn Thr Arg Tyr
           200                 205                 210
   TTT GGG ACA CTA ACA GGA GGA GAA AAT GTT CCT CCA GTT CTT CAT ATA        725
   Phe Gly Thr Leu Thr Gly Gly Glu Asn Val Pro Pro Val Leu His Ile
       215                 220                 225
   ACA AAC ACT GCC ACA ACA GTG TTG CTT GAT GAA TTT GGT GTT GGG CCA        773
   Thr Asn Thr Ala Thr Thr Val Leu Leu Asp Glu Phe Gly Val Gly Pro
   230                 235                 240                 245
   CTT TGC AAA GGT GAC AAC TTA TAC TTG TCA GCT GTT GAT GTC TGT GGC        821
   Leu Cys Lys Gly Asp Asn Leu Tyr Leu Ser Ala Val Asp Val Cys Gly
                   250                 255                 260
   ATG TTT ACA AAC AGG TCT GGT TCC CAG CAG TGG AGA GGA CTC TCC AGA        869
   Met Phe Thr Asn Arg Ser Gly Ser Gln Gln Trp Arg Gly Leu Ser Arg
               265                 270                 275
   TAT TTT AAG GTG CAG CTA AGG AAA AGG AGG GTT AAA AAC CCC TAC CCA        917
   Tyr Phe Lys Val Gln Leu Arg Lys Arg Arg Val Lys Asn Pro Tyr Pro
           280                 285                 290
   ATT TCT TTC CTT CTT ACT GAT TTA ATT AAC AGA AGG ACT CCT AGA GTT        965
   Ile Ser Phe Leu Leu Thr Asp Leu Ile Asn Arg Arg Thr Pro Arg Val
       295                 300                 305
   GAT GGG CAG CCT ATG TAT GGC ATG GAT GCT CAA GTA GAG GAG GTT AGA      1013
   Asp Gly Gln Pro Met Tyr Gly Met Asp Ala Gln Val Glu Glu Val Arg
   310                 315                 320                 325
   GTT TTT GAG GGA ACA GAG GAG CTT CCA GGG GAC CCA GAC ATG ATG AGA      1061
   Val Phe Glu Gly Thr Glu Glu Leu Pro Gly Asp Pro Asp Met Met Arg
                   330                 335                 340
   TAC GTT GAC AAA TAT GGA CAG TTG CAG ACA AAA ATG CTG TAATCAAAAG       1110
   Tyr Val Asp Lys Tyr Gly Gln Leu Gln Thr Lys Met Leu
               345                 350
   CTTTTATTGT A                                                         1121
(2) ANGABEN ZU SEQ ID NO: 2:
   (i) SEQUENZKENNZEICHEN:
      (A) LÄNGE: 354 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Ala Pro Thr Lys Arg Lys Gly Glu Arg Lys Asp Pro Val Gln Val
    1               5                  10                  15
   Pro Lys Leu Leu Ile Arg Gly Gly Val Glu Val Leu Glu Val Lys Thr
               20                  25                  30
   Gly Val Asp Ser Ile Thr Glu Val Glu Cys Phe Leu Thr Pro Glu Met
           35                  40                  45
   Gly Asp Pro Asp Glu His Leu Arg Gly Phe Ser Lys Ser Ile Ser Ile
       50                  55                  60
   Ser Asp Thr Phe Glu Ser Asp Ser Pro Asn Arg Asp Met Leu Pro Cys
   65                  70                  75                  80
   Tyr Ser Val Ala Arg Ile Pro Leu Pro Asn Leu Asn Glu Asp Leu Thr
                   85                  90                  95
   Cys Gly Asn Ile Leu Met Trp Glu Ala Val Thr Leu Lys Thr Glu Val
               100                 105                 110
   Ile Gly Val Thr Ser Leu Met Asn Val His Ser Asn Gly Gln Ala Thr
           115                 120                 125
   His Asp Asn Gly Ala Gly Lys Pro Val Gln Gly Thr Ser Phe His Phe
       130                 135                 140
   Phe Ser Val Gly Gly Glu Ala Leu Glu Leu Gln Gly Val Leu Phe Asn
   145                 150                 155                 160
   Tyr Arg Thr Lys Tyr Pro Asp Gly Thr Ile Phe Pro Lys Asn Ala Thr
                   165                 170                 175
   Val Gln Ser Gln Val Met Asn Thr Glu His Lys Ala Tyr Leu Asp Lys
               180                 185                 190
   Asn Lys Ala Tyr Pro Val Glu Cys Trp Val Pro Asp Pro Thr Arg Asn
           195                 200                 205
   Glu Asn Thr Arg Tyr Phe Gly Thr Leu Thr Gly Gly Glu Asn Val Pro
       210                 215                 220
   Pro Val Leu His Ile Thr Asn Thr Ala Thr Thr Val Leu Leu Asp Glu
   225                 230                 235                 240
   Phe Gly Val Gly Pro Leu Cys Lys Gly Asp Asn Leu Tyr Leu Ser Ala
                   245                 250                 255
   Val Asp Val Cys Gly Met Phe Thr Asn Arg Ser Gly Ser Gln Gln Trp
               260                 265                 270
   Arg Gly Leu Ser Arg Tyr Phe Lys Val Gln Leu Arg Lys Arg Arg Val
           275                 280                 285
   Lys Asn Pro Tyr Pro Ile Ser Phe Leu Leu Thr Asp Leu Ile Asn Arg
       290                 295                 300
   Arg Thr Pro Arg Val Asp Gly Gln Pro Met Tyr Gly Met Asp Ala Gln
   305                 310                 315                 320
   Val Glu Glu Val Arg Val Phe Glu Gly Thr Glu Glu Leu Pro Gly Asp
                   325                 330                 335
   Pro Asp Met Met Arg Tyr Val Asp Lys Tyr Gly Gln Leu Gln Thr Lys
               340                 345                 350
   Met Leu
(2) ANGABEN ZU SEQ ID NO: 3:
   (i) SEQUENZKENNZEICHEN:
      (A) LÄNGE: 29 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 3:
   GTACGGGACT GCAGCACCTG CTCTTGAAG 29
(2) ANGABEN ZU SEQ ID NO: 4:
   (i) SEQUENZKENNZEICHEN:
      (A) LÄNGE: 29 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 4:
   TACAATAAAA GCTTTTGATT ACAGCATTT 29

## Beschreibung

Die vorliegende Erfindung betrifft neue Virus like particles (Virus-ähnliche Teilchen) und deren Verwendung in analytischen, diagnostischen und therapeutischen Verfahren.

JC-Virus (JCV) gehört der Gruppe der humanen Polyomaviren an. JCV kann eine subakute demyelinisierende Erkrankung des Gehirns durch eine lytische Infektion der Myelin-bildenden Oligodendrozyten und eine abortive Infektion von Astrozyten hervorrufen. Diese Infektion, die klinisch als progressive multifokale Leukoenzephalopathie (PML) bezeichnet wird, führt zum Entstehen von Demyelinisierungsfoci im Cerebrum cerebellum und Stammhirn und endet üblicherweise innerhalb weniger Monate lethal.

Bei PML tritt üblicherweise keine signifikante humorale oder zelluläre Immunantwort gegen JCV auf, wodurch eine Diagnose der Erkrankung erschwert wird. Obwohl JCV in etwa 80 % der erwachsenen Bevölkerung vorhanden zu sein scheint, entwickelt sich die PML im allgemeinen nur im Zusammenhang mit einer Schwächung des Immunsystems. Die zunehmende Verwendung von immunsuppressiven Arzneimitteln und die steigende Anzahl HIVinfizierter Patienten führten in den vergangenen Jahren zu einem beträchtlichen Anstieg von PML-Erkrankungen. Nach derzeitigen Schätzungen entwickelt sich eine PML in etwa 2-5 % der AIDS-Patienten.

Die bekannten Diagnoseverfahren zum Nachweis einer PML-Erkrankung umfassen im wesentlichen bildgebende Verfahren wie etwa CT (Computertomographie) und MRI (Magnetic Resonance Imaging), sowie immuncytochemische Verfahren anhand von Biopsien bzw. Autopsien. In jüngerer Zeit haben PCR-Nachweisverfahren zunehmend an Bedeutung gewonnen, wobei die Virus-DNA-Amplifikation aus Cerebrospinalflüssigkeit (CSF) zuverlässige und spezifische Ergebnisse liefert (Weber et al., J. Infect. Dis. (1994), 1138-1141 und Mc Guire et al., Annals of Neurology 37 (1995), 395-399).

Nachteile der aus dem Stand der Technik bekannten Diagnoseverfahren bestehen darin, daß sie nur mit hohem Aufwand durchführbar und daher für eine zuverlässige Routinediagnostik ungeeignet sind. Dies gilt auch für die PCR-Verfahren, bei denen ein hohes Kontaminationsrisiko besteht, das zur Verfälschung von Ergebnissen führen kann. Es wäre daher wünschenswert, über ein Verfahren und Reagenzien zu verfügen, die auf einfachere Weise eine zuverlässige Diagnose von PML gestatten. Darüberhinaus besteht ein Bedarf nach einem Mittel, das zur therapeutischen Behandlung bei PML-Erkrankungen eingesetzt werden kann.

Die der Erfindung zugrundeliegende Aufgabe besteht somit darin, Verfahren und Reagenzien bereitzustellen, mit denen die oben genannten Ziele erreicht und die Nachteile des Standes der Technik mindestens teilweise vermieden werden können.

Überraschenderweise wurde festgestellt, daß das Virusprotein 1 (VP1) von JC-Virus ein hervorragendes Mittel zum immunchemischen Nachweis von JCV darstellt. Darüberhinaus wurde gefunden, daß VP1 in großer Ausbeute in Form von Virus-ähnlichen Partikeln (VLP) durch rekombinante DNA-Techniken herstellbar ist.

Ein Gegenstand der Erfindung ist somit ein Virus-ähnliches Partikel (VLP), das aus mehreren Molekülen des Virusprotein 1 (VP1), dem Hauptstrukturprotein von JC-Virus aufgebaut ist. Dieses VLP zeichnet sich insbesondere dadurch aus, daß es eine immunologisch mit Anti-JCV-Antiseren reagierende Struktur aufweist und frei von mit JCV assoziierten Nukleinsäuren ist.

Es wurde überraschenderweise festgestellt, daß rekombinantes VP1 von JVC nach Reinigung zu Virus-ähnlichen Partikeln (VLP) assoziieren kann. Derartige VLP zeigen eine ikosaedrische Struktur mit einem Durchmesser von etwa 50 nm. Der Vorteil der VLP gegenüber einem einzelnen VP1 Protein ist vor allem darin zu sehen, daß die Eigenschaften oder/und Wirkungen des natürlichen Virus genauer simuliert werden und daß sich ein breiteres Anwendungsgebiet erschließt. Gegenüber dem natürlichen Virus sind weitere Vorteile insbesondere in der erhöhten Arbeitssicherheit zu sehen und überraschenderweise wurde auch festgestellt, daß das VLP im Vergleich zum gereinigten Gesamtvirus eine erhöhte Immunogenität aufweist.

Das VP1 von JVC ist das Hauptstrukturprotein der Kapsidhülle von JCV, z.B. aus Wildtypstämmen oder mutagenisierten Stämmen von JCV. In einer besonderen Ausführungsform ist das VLP aus rekombinant hergestelltem VP1 aufgebaut. In diesem Fall umfaßt der Begriff VP1 auch Proteine, die sich von Wildtyp VP1 durch Mutationen wie etwa Substitutionen, Insertionen oder/und Deletionen unterscheiden. Zur Herstellung von rekombinanten VP1 verwendet man bevorzugt eine Nukleinsäure, welche die in SEQ ID NO. 1 gezeigte Sequenz, eine dieser Sequenz im Rahmen der Degeneration des genetischen Codes entsprechende Sequenz oder eine damit unter stringenten Bedingungen hybridisierende Sequenz umfaßt, wobei man die Nukleinsäuresequenz oder einen diese Sequenz enthaltenden rekombinanten Vektor in eine geeignete Wirtszelle einbringt, die Wirtszelle unter Bedingungen kultiviert, bei denen eine Expression der Nukleinsäuresequenz erfolgt und das Protein aus der Zelle oder dem Zellüberstand isoliert. Stringente Hybridisierungsbedingungen sind vorzugsweise definiert gemäß Sambrook et al. (1989) Molecular Cloning A Laboratary Manual, Second Edition, Cold Spring Harbor Laboratory Press, und umfassen einen Waschschritt von 30 min in 0,1 x SSC, 0,5% SDS bei 68°C.

Das erfindungsgemäße VLP kann weiterhin ein oder mehrere zusätzliche heterologe Proteine in der Kapsidstruktur aufweisen. Darunter ist zu verstehen, daß ein heterologes Protein in der Kapsidstruktur verankert ist, wobei bevorzugt mindestens ein Teil dieses Proteins von außen zugänglich ist. Als heterologes Protein eignen sich hierfür prinzipiell alle Proteine, die in die Kapsidstruktur inkorporiert werden können und die Selbstassemblierung des VLP nicht beeinträchtigen. Beispielsweise kann es wünschenswert sein, das VLP mittels des heterologen Proteins mit einer antigenen Determinante zu versehen, die durch einen spezifischen Antikörper nachgewiesen werden kann. Andererseits kann das heterologe Protein ein Bindungspartner für einen Zelloberflächenrezeptor sein, wodurch eine Interaktion des VLP mit einer spezifischen Klasse oder Subklasse von Zellen, die den entsprechenden Rezeptor tragen, möglich ist. Eine derartige Interaktion ist beispielsweise nach Bindung an den Rezeptor das Auslösen eines bestimmten Signals oder die Internalisierung des VLP.

Die Herkunft der Proteine ist nicht auf einen bestimmten Organismus beschränkt und in Abhängigkeit der besonderen Anwendungsform können eukaryontische, insbesondere humane Proteine, oder auch prokaryontische oder virale Proteine in das VLP inkorporiert werden. Beispiele für geeignete Proteine sind oberflächenproteine bzw. Membranproteine und exemplarisch seien CD4 für einen humanen Zelloberflächenrezeptor und das Hüllprotein eines Immundefizienzvirus, z.B. HIV-gp 120, für ein virales Oberflächenprotein angeführt. Es versteht sich, daß das heterologe Protein gegebenenfalls unter Verwendung rekombinanter Methoden verändert, z.B. verkürzt werden kann, insbesondere falls das natürliche Protein nicht in das VLP inkorporiert werden kann oder die Selbstassemblierung beeinträchtigt.

In einer weiteren besonderen Ausführungsform kann das VLP im Inneren der Kapsidstruktur eine oder mehrere Wirksubstanzen enthalten. Unter Wirksubstanz wird in dieser Beschreibung jedes Molekül verstanden, welches in dem bei der Selbstassemblierung verwendeten Medium nicht üblicherweise vorhanden ist. Solche Wirksubstanzen umfassen z.B. Makromoleküle wie etwa Nukleinsäuren, d.h. RNA, DNA oder artifizielle modifizierte Nukleinsäuren, sowie Proteine und andere physiologisch aktive Stoffe, die natürlicher, synthetischer oder rekombinanter Art sein können. Beispiele für derartige physiologisch aktive Stoffe sind z.B. Lipide, Phospholipide, Peptide, Arzneimittel, Toxine etc.

Noch ein weiterer Gegenstand der vorliegenden Erfindung ist eine Nukleinsäure, die für ein VP1 Protein codiert und
(a) die SEQ ID NO. 1 gezeigte Nukleotidsequenz,
(b) eine der Sequenz aus (a) im Rahmen der Degeneration des genetischen Codes entsprechende Nukleotidsequenz oder/und
(c) eine mit einer der Sequenzen au (a) oder/und (b) unter stringenten Bedingungen hybridisierende Nukleotidsequenz umfaßt.

Die erfindungsgemäße Nukleinsäure ist vorzugsweise auf einem rekombinanten Vektor, insbesondere unter Kontrolle eines Expressionssignals lokalisiert. Beispiele geeigneter Vektoren sind bei Sambrook et al., Supra, Kapitel 1, 2, 3, 4, 16 und 17 beschrieben. Die Erfindung betrifft auch eine Zelle, die mit einem erfindungsgemäßen Vektor transformiert ist.

In einem anderen Aspekt betrifft die Erfindung auch ein Verfahren zur Herstellung eines VLP, wobei man VP1 aufreinigt und in eine Form überführt, bei denen Assemblierung mehrerer VP1-Moleküle zu einem VLP erfolgt. Geeignete Bedingungen für eine Assemblierung liegen beispielsweise bei der Reinigung von VP1 aus Zellkulturüberstand nach einer differentiellen Kissenzentrifugation über Sucrose und Metrizamid vor.

Bevorzugt wird das VLP auf rekombinante Weise hergestellt, wobei man eine für ein VP1-Protein codierende Nukleinsäure in eine Zelle einbringt, die transformierte Zelle in einem Medium unter Bedingungen kultiviert, bei denen eine Expression der Nukleinsäure erfolgt, und das Expressionsprodukt aus der Zelle oder aus dem Medium gewinnt. Die Isolierung des rekombinanten VP1 erfolgt in Abhängigkeit des verwendeten Wirt/Vektorsystems direkt aus den Wirtszellen oder/und dem Zellkulturüberstand.

Der Vorteil des rekombinanten Verfahrens liegt vor allem darin, daß auf einfache Weise VLP in hoher Reinheit und in großen Mengen gewonnen werden können. Als Expressionssystem hat sich in der Praxis die Verwendung von Baculoviren in Verbindung mit Insektenzellen, z.B. mit der Insektenzellinie Sf 158 bewährt.

Zur Herstellung von VLP, die innerhalb der Kapsidstruktur ein heterologes Protein inkorporiert haben, bzw. von VLP, die im Inneren der Kapsidstruktur eine Wirksubstanz enthalten, modifiziert man das obige Herstellungsverfahren, indem man zu einem geeigneten Zeitpunkt, d.h. vor der Assemblierung der VLP die heterologen Proteine oder/und Wirksubstanzen in der gewünschten Menge bzw. Konzentration zusetzt und anschließend die Assemblierung zuläßt. Auf diese Weise können VLP entstehen, welche heterologes Protein der Kapsidhülle inkorporiert haben oder/und im Inneren eine eingeschlossene Wirksubstanz, z.B. eine Nucleinsäure enthalten. Die Inkorporation von heterologen Polypeptiden in der Kapsidhülle kann beispielsweise durch rekombinante Koexpression der jeweiligen Polypeptide, d.h. des VP1-Polypeptids und des heterologen Polypeptids in einer geeigneten Wirtszelle, z.B. einer eukaryontischen Zelle, erfolgen. Die Inkorporation von Wirksubstanzen in das Innere der Kapsidhülle kann z.B. durch Dissoziation der Kapsidhülle und anschließende Reassoziation in Gegenwart der Wirksubstanz oder durch osmotischen Schock der VLP in Gegenwart der Wirksubstanz erfolgen.

In einem nochmals weiteren Aspekt betrifft die vorliegende Erfindung ein Verfahren zur immunologischen Bestimmung spezifischer Antikörper gegen JCV in einer Probe, wobei man die Antikörper durch Bindung an ein aus VP1 aufgebautes VLP oder einen Bestandteil davon qualitativ oder/und quantitativ nachweist. Aus Gründen der einfachen Verfügbarkeit ist die Verwendung von aus rekombinanten VP1-Molekülen aufgebauten VLP bevorzugt.

Testformate für derartige immunologische Bestimmungsverfahren sind dem Fachmann in großer Anzahl bekannt und müssen daher nicht im einzelnen erläutert werden. Im allgemeinen werden derartige Verfahren so durchgeführt, daß man die VLP bzw. deren Bestandteile mit der Probe unter geeigneten Bedingungen in Kontakt bringt, um die Bindung spezifischer Antikörper gegen JC-Virus an die VLP bzw. deren Bestandteile zu gestatten, die gebildeten Immunkomplexe von anderen Probenbestandteilen abtrennt und die Gegenwart von Antikörpern nachweist. Hierzu kann man beispielsweise VLP verwenden, die adsorptiv, kovalent oder über einen geeigneten Bindungspartner an eine Festphase gekoppelt sind.

Der nach Inkubation mit der Probeflüssigkeit an das Virus-Protein gebundene nachzuweisende Antikörper wird anschließend durch einen spezifischen, gegen den nachzuweisenden Antikörper gerichteten Rezeptor, z.B. einen Detektionsantikörper, Protein A oder ein mit dem Antikörper bindefähiges Antigen, z.B. ein VLP detektiert. Die genaue Art dieses Rezeptors ist nicht kritisch und es können z.B. polyklonale oder monoklonale Nachweis-Antikörper verwendet werden, wie beispielsweise Antihuman-Antikörper aus diversen Tierarten, wie etwa Kaninchen, Maus oder Ziege, oder Antikörper, die spezifisch gegen den Fc-Teil von Immunglobulinen oder Immunglobulinklassen gerichtet sind.

Der mit dem nachzuweisenden Antikörper bindefähige Rezeptor umfaßt weiterhin ein Mittel, das die Detektion gestattet, d.h. eine Markierung die direkt oder indirekt, z.B. mittels spezifischer Bindungspaare, wie etwa Biotin/Avidin, mit dem Rezeptor assoziiert ist. Derartige Markierungen sind dem Fachmann ebenfalls bekannt und umfassen etwa Radioaktivität, Enzyme, Lumineszenz- oder Fluoreszenzmarkierungen. Ein besonders bevorzugtes Testformat ist der ELISA, bei dem ein Rezeptor mit Enzymmarkierung eingesetzt wird.

Das obige immunologische Bestimmungsverfahren kann zur Diagnose von progressiver multifokaler Leukoenzephalopathie (PML) eingesetzt werden, wenn man als Probe Cerebrospinalflüssigkeit (CSF) verwendet. In einer bevorzugten Ausführungsform führt man zur Diagnose von PML eine Parallelbestimmung durch, wobei als Proben sowohl CSF als auch Serum aus der gleichen Person verwendet werden, wobei diese beiden Proben besonders bevorzugt parallel entnommen werden. Um aussagekräftige Ergebnisse zu erhalten, ist es besonders bevorzugt, die gemessenen Werte auf die Gesamtimmunglobulinkonzentration zu normieren. Eine geeignete Bezugsgröße ist der sogenannte Antikörperspezifitätsindex (ASI), welcher definiert ist, als das Verhältnis des Titers spezifischer Antikörper gegen JCV in CSF und Serum geteilt durch das Verhältnis der Ig-Gesamtkonzentration in CSF und Serum. Es hat sich herausgestellt, daß ein ASI-Wert von ≥ 1,5 für eine positive Diagnose von PML zuverlässige Ergebnisse ergibt.

Ein weiterer Gegenstand der vorliegenden Erfindung ist somit ein Testkit zur Bestimmung spezifischer Antikörper gegen JCV, der in getrennter räumlicher Anordnung VLP von JCV oder Bestandteile davon, Mittel zum Nachweis von Antikörpern und gegebenenfalls übliche Puffer und Hilfssubstanzen umfaßt. Vorzugsweise eignet sich der Testkit zum Nachweis humaner Antikörper. Das Mittel zum Nachweis von Antikörpern umfaßt insbesondere einen spezifisch mit dem nachzuweisenden Antikörper bindefähigen Rezeptor, der direkt oder indirekt mit einer detektierbaren Markierung versehen ist. Der Testkit kann ebenfalls die erforderlichen Substrate bzw. Detektionssubstanzen enthalten.

Die erfindungsgemäßen VLP können auch therapeutisch eingesetzt werden, z.B. zur Behandlung von PML. So eigenen sich die erfindungsgemäßen VLP zur Herstellung eines therapeutischen oder/und prophylaktischen Impfstoffes, der gegen eine Infektion mit JVC eingesetzt wird. Diesbezüglich ist insbesondere vorgesehen, ein VLP zu verwenden, welches weder heterologe Proteine der Kapsidstruktur inkorporiert noch im Innern Wirksubstanzen eingeschlossen hat. Dadurch können einerseits Rezeptoren auf der Oberfläche der Oligodendrozyten abgesättigt werden, um die weitere Infektion zumindest zu verlangsamen und andererseits die humorale Immunantwort gegen JCV zu stimulieren. Auch die zelluläre Immunantwort, die sich durch eine Antigen-spezifische T-Zellproliferation nachweisen läßt, kann durch Verabreichung von rekombinantem VP1 stimuliert werden. Alternativ können jedoch auch modifizierte VLP verwendet werden, welche dann bevorzugt in der Kapsidstruktur einen Bindungspartner für einen Oberflächenrezeptor der entsprechenden Zielzellen, also insbesondere von Oligodendrozyten inkorporiert haben und im Inneren mindestens eine Wirksubstanz eingeschlossen haben, welche die Vermehrung oder Verbreitung des Virus behindert. In diesem Fall ist die Wirksubstanz bevorzugt ein Virostatikum, wie etwa Cytosinarabinosid.

In einer weiteren Ausführungsform kann man die VLP als Transportvehikel verwenden. Derartige VLP enthalten in ihrem Inneren eingeschlossen eine geeignete Wirksubstanz und bevorzugt ein heterologes in die Kapsidstruktur inkorporiertes Protein, das mit einem spezifischen Oberflächenrezeptor der beabsichtigten Zielzelle bindungsfähig ist. Auf diese Weise kann die Spezifität der Interaktion mit den vorgesehenen Zielzellen gewährleistet und in Abhängigkeit von der Anwendung auf eine Vielzahl an Zelltypen angepaßt werden. Ein Beispiel für eine derartige Verwendung ist der zielgerichtete Transport von TNF-α Antisense-Nukleinsäuren an Oligodendrozyten bei Multipler Sklerose, da bekannt ist, daß bei dieser Erkrankung eine TNF-α Expression im Schub zur Demyelinisierung führt. Ein anderes Beispiel ist die Verwendung von VLP als Transportersystem für Nucleinsäuren in der Gentherapie.

Ein weiterer Gegenstand der Erfindung ist somit eine pharmazeutische Zusammensetzung umfassend ein VLP, sowie gegebenenfalls übliche Puffer, Hilfs-, Zusatz- oder/und Verdünnungsmittel. Bei dem VLP kann es sich hierbei um ein unmodifiziertes VLP handeln oder um ein VLP, welches in der Kapsidstruktur ein heterologes Protein inkorporiert oder/und im Inneren eine Wirksubstanz eingeschlossen hat.

Die Erfindung wird nunmehr weiter erläutert durch die nachfolgenden Beispiele sowie die beigefügten Figuren und Sequenzprotokolle. Es zeigen:
- SEQ ID NO. 1: die Nucleotidsequenz einer aus JCV isolierten DNA-Sequenz, die für VP1 codiert;
- SEQ ID NO. 2: die korrespondierende Aminosäuresequenz;
- SEQ ID NO. 3: einen zur Isolierung der VP1 codierenden Sequenz verwendeten Amplifikationsprimer;
- SEQ ID NO. 4: einen weiteren Amplifikationsprimer und
- Figur 1: eine schematische Darstellung der Arbeitsschritte zur Herstellung von rekombinantem VP1,
- Figur 2: die Verpackung von heterologer DNA in VP1-VLP,
- Figur 3: die Immunreaktivität eines Anti-VP1-VLP-Immunserums und
- Figur 4: die Neutralisation von JCV durch Anti-VP1-VLP-Immunserum.

### BEISPIELE

### Beispiel 1:

### Rekombinante Herstellung von JCV-VP1

JCV-DNA (Stamm Mad-4) wurde aus SVG-Zellen isoliert und die kodierende Sequenz des VP1-Gens mittels PCR amplifiziert. Für die PCR wurden die nachstehenden Primer verwendet, die zur anschließenden Klonierung mit einer BamHI bzw. HindIII Restriktionsschnittstelle versehen sind.

In der PCR-Reaktion wurden in einem Reaktionsvolumen von 50 µl jeweils 100 pmol Primer eingesetzt. Die DNA wurde in einem 50 µl Gemisch durch 20 Amplifikationszyklen, bestehend aus 45 s Denaturierung bei 94°C, 45 s Annealing bei 55°C und 45 s Extension bei 70°C voramplifiziert. Im ersten Zyklus wurde die Denaturierung auf 5 min verlängert. Jede nachfolgende Extension wurde um 1 s und der letzte Zyklus auf 5 min verlängert. Aus dieser Reaktion wurden 10 µl mit den Primern und 2.0 U Taq DNA Polymerase (Pharmacia, Freiburg, Deutschland) vermischt und einer PCR unter den obigen Bedingungen für insgesamt 25 Zyklen unterzogen.

Die PCR-Produkte wurden über Agarosegelelektrophorese gereinigt, mit BamHI und HindIII gespalten und in die Klonierungsstelle eines BamHI/HindIII geschnittenen p-BlueBac-III-Vektors (Invitrogen, Niederlande) ligiert, wobei der das VP1-Gen enthaltende Vektor pBlueBac-VP halten wird. Nach Transformation von E.coli Zellen mit pBlueBac-VP und Identifizierung positiver Klone wurden die rekombinanten Plasmide isoliert und mit Wildtyp Baculovirus DNA in Sf 158 Insektenzellen transfiziert.

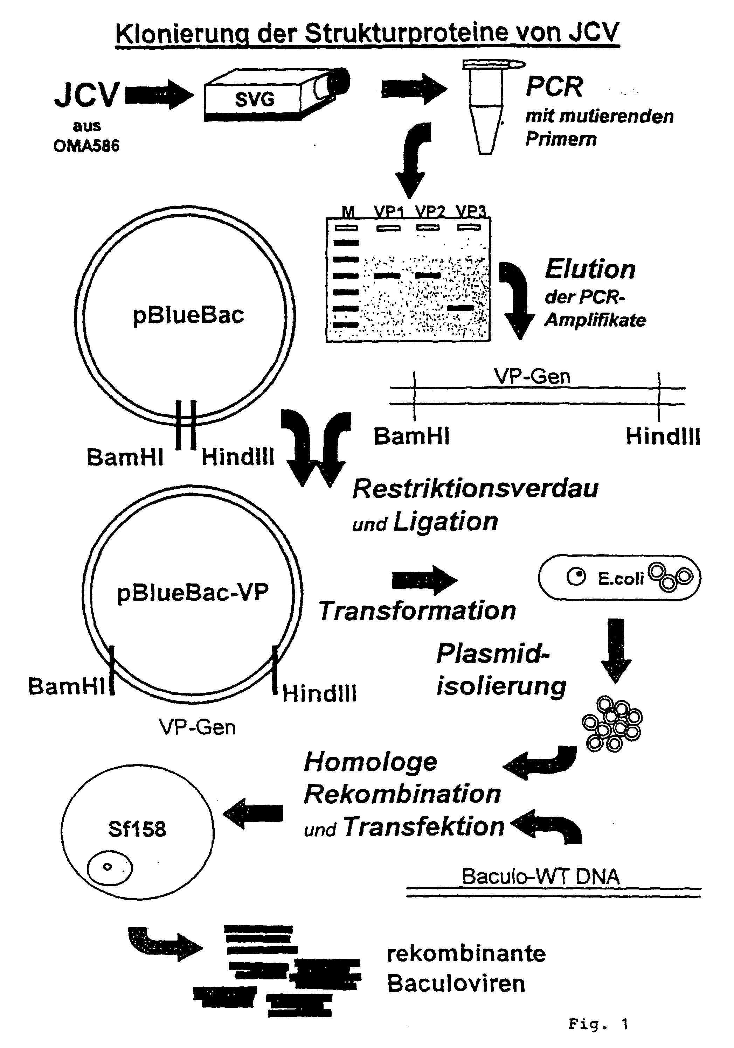

Nach zweimaliger Passage des Überstandes auf Sf 158-Zellen wurden die rekombinanten Baculoviren gereinigt (vgl. Fig. 1).

Das rekombinante VP1 wurde in den Sf 158 Wirtszellen exprimiert und in einer Konzentration von 0,9 mg Protein pro ml in den Zellüberstand sekretiert. Der Nachweis der Expression erfolgte durch Western-Blot von Zellkulturüberständen unter Verwendung eines Kaninchen-anti-SV40-Hyperimmunserums.

Aus dem Zellkulturüberstand konnte das VP1 mittels differenzieller Kissenzentrifugation über Sucrose und Metrizamid bis zur Homogenität gereinigt werden.

Durch elektronenmikroskopische Untersuchungen kann gezeigt werden, daß das VP1 zu VLP assoziierte, die einen ikosaedrischen Aufbau mit einem Durchmesser von etwa 50 nm und im CsCl-Gradienten eine Dichte von ca. 1,31 g/ml aufweisen. Proteinbiochemische Untersuchungen des in den VLP-enthaltenden VP1 mittels Massenspektroskopie nach tryptischen Verdau gab die erwarteten Fragmentmassen. Die rekombinanten VLP erwiesen sich nach Immunisierung im Kaninchen als hoch immunogen.

### Beispiel 2:

### Immunologische Bestimmung spezifischer Antikörper gegen JC-Virus in Serum und CSF

Rekombinantes VP1 wurde gegen das Hyperimmunserum gegen SV40 aus Kaninchen und Antiseren von PML-Patienten austitriert. Die optimale Antigenkonzentration wurde mit 200 ng Protein des nach differenzieller Kissenzentrifugation erhaltenen Produkts entsprechend - 70 ng VP1 pro Vertiefung ermittelt. CSF Gesamtprotein wurde mittels Trichloressigsäurepräzipitation bestimmt. Immunglobulin G und Albumin in Serum und CSF wurden durch immunochemisch durch Nephelometrie bestimmt. Die intrathekale Antikörpersynthese wurde gemäß den Methoden von Reiber und Lange (Clin. Chem. 37 (1991), 1153 - 1160) und Weber et al. (J.

Immunol. Methods 136 (1991), 133 - 137) analysiert. Die intrathekale Synthese VP1-spezifischer Antikörper wurde als ein Verhältnis der Antikörpertiter in CSF und Serum (ASI) von mindestens 1,5 definiert.

Zur Durchführung der Bestimmung wurden 200 ng des Zellkulturüberstandes von mit pBlueBac-III-VP1 infizierten Sf 158-Zellen pro Vertiefung einer Mikrotiterplatte mit 96 Vertiefungen (Grainer Frickenhausen, Deutschland) bei 4°C, während 12 bis 14 Stunden aufgetragen. Die unspezifische Bindung wurde durch Inkubation mit 200 µl 5% Blotto pro Vertiefung bei 37°C während 1 Stunde abgesättigt. Serum wurde in Blotto verdünnt, ausgehend von 1 : 500 in Log2-Schritten, wobei die höchste verwendete Verdünnung 1 zu 80000 war. CSF wurde in Blotto verdünnt, ausgehend von 1 : 5 in Log2-Schritten, wobei die höchste verwendete Verdünnung 1 zu 40000 betrug. Nach Bindung der primären Antikörper während 3 Stunden bei 37°C wurden die Platten 7 mal mit 150 mM PBS mit 0,05 % Tween 20 gewaschen. Gebundener Antikörper wurde durch Inkubation mit einem Peroxidase-konjugierten Anti-Human-IgG-Antiserum aus Kaninchen (Jackson Laboratories Dianova, Hamburg, Deutschland) für 2 Stunden bei 37°C nachgewiesen. Nach 7 Waschungen mit PBS wurde der Peroxidase-konjugierte Antikörper unter Verwendung von o-Phenylendiamin sichtbar gemacht. Nach 15 Minuten wurde die Reaktion mit 1,3 N Schwefelsäure gestoppt und die optische Dichte (OD) in einem Titertek MC340 MKII-ELISA-Lesegerät bei 495/620 nm gemessen. Die Antikörpertiter in CSF und Serum wurden unter Verwendung von EXEL™ berechnet.

In ausgewählten Fällen wurden eine Western Blot-Analyse der intrathekalen Immunantwort durchgeführt. Nach elektrophoretischer Auftrennung von 1µg VP1 pro Bahn in einem 12 % SDS-PAGE-Gel wurde das Antigen elektrophoretisch auf eine HiBond-PVDF-Membran (Amersham Buchler KG, Deutschland) transferiert. Die unspezifische Antikörperbindung wurde mit 5 % Blotto für 1 Stunde bei 37°C abgesättigt. Serum- oder CSF-Antikörper wurden in einer IgG-Konzentration von 10 mg/l zu jeder Bahn zugegeben und 3 Stunden bei 37°C inkubiert. Nach 3 Waschungen in PBS wurden die Membranen mit einem Peroxidase-konjugierten Anti-Human-IgG-Antiserum aus Kaninchen (Jackson Laboratories Dianova, Hamburg) inkubiert. Gebundene Nachweisantikörper wurden unter Verwendung des ECL-Kits (Amersham Buchler KG, Deutschland) gemäß den Herstellerangaben auf Hyperfilm-ECL sichtbar gemacht.

### Beispiel 3:

### Diagnostische Bestimmung von PML

Parallele CSF/Serumproben von 189 Patienten wurden in die Untersuchung aufgenommen. In 28/34 (82 %) der PML-Patienten wurde bei einem ASI ≥ 1,5 die intrathekale Synthese von Anti-VP1 Antikörpern festgestellt, während ein ASI ≥ 1,5 in nur 5/155 Kontrollpatienten gefunden wurde. Serumantikörper gegen VP1 wurden in allen PML-Patienten sowie in 28/37 (76 %) der Multiple Sklerose-Patienten, 43/50 (86%) der Kontrollgruppe, 29/33 (88 %) der Patienten mit gestörter Bluthirnschrankenfunktion und 31/35 (89 %) der HIV-positiven Patienten festgestellt.

In Einzelfällen wurde die intrathekale Synthese von Anti-VP1 Antikörpern durch Western Blot-Analyse überprüft. Bei PML-Patienten mit einem VP1 ASI < 1,5 wurde in CSF und Serum eine annähernd gleiche Intensität erhalten, während 4 Seren von Patienten mit ASI im Bereich von 12-107 sehr schwache Serumbanden und deutlich stärkere CSF-Banden zeigten.

Es wurde eine statistische Auswertung der Antikörperspezifitätsindizes (ASI) in den 5 unterschiedlichen Patientengruppen durchgeführt. In der Normalgruppe betrug der mittlere VP1 ASI 0,92 (± 0,16, Bereich 0,65 - 1,26), in der Multiple Sklerose-Gruppe betrug er 0,91 (± 0,38, Bereich 0,34 - 2,49), in der Gruppe mit gestörter Bluthirnschrankenfunktion betrug er 0,92 (± 0,22, Bereich 0,52 - 1,36), in der HIV-positiven Gruppe betrug er 4,35 (± 12,7, Bereich 0,35 - 67,2) und in der PML-Gruppe betrug er 12,59 (± 24,42, Bereich 0,38 - 107). Bei Verwendung des Kruskal-Wallis-Test gibt es einen signifikanten Unterschied zwischen den 5 Gruppen (P < 0,0001). Bei Verwendung des Mann-Whitney-Tests gibt es einen signifikanten Unterschied zwischen der PML-Gruppe und der Kontrollgruppe, der Gruppe mit gestörter Bluthirnschrankenfunktion und der Multiple Sklerose-Gruppe (P < 0,0001) und zwischen der PML-Gruppe und der HIV-positiven Gruppe bei P < 0,001. Die Unterschiede zwischen den anderen 6 Gruppenpaaren waren nicht signifikant.

Die obigen Ergebnisse zeigen, daß eine Untersuchung der intrathekalen Synthese von gegen JCV-VP1 gerichteten Antikörpern ein geeigneter Test für die Diagnose von PML darstellt. In der durchgeführten Untersuchung wurde unter Verwendung eines statistisch signifikanten VP1-ASI von ≥ 1,5 eine Sensitivität von 82 % und eine Spezifität von 96 % festgestellt. Obwohl der ELISA-Assay somit eine etwas geringere Sensitivität als ein PCR-Assay mit nested Primern (93 %) aufweist, stellt er, da kostengünstiger, einfacher durchzuführen und weniger anfällig für falsch-positive Resultate durch Kontamination eine sinnvolle Ergänzung bestehender Testverfahren dar. Wichtige Anwendungen umfassen das Screening und Untersuchungen mit einer Vielzahl von Proben, die schnelle Bestimmung eindeutig positiver Proben, sowie die Aussonderung eindeutig negativer Proben, da nach den bisher vorliegenden Ergebnissen ein negativer Antikörpertest in Serum die Diagnose von PML ausschließt.

### Beispiel 4 :

### Nachweis einer zellulären Immunantwort gegen rekombinantes VP1

Bei einem gesunden Probanden und einem PML-Patienten konnte eine zelluläre Immunantwort gegen rekombinantes JCV-VP1 nachgewiesen werden. Die Antigen-spezifische Proliferation war dosisabhängig. Bei einer Endkonzentration von 1 - 10 µg Antigen wurde eine Antigen-spezifische T-Zellproliferation nachgewiesen, die am 6. Tag ihr Maximum erreichte. Dieser Versuch weist darauf hin, daß gesunde Probanden und PML-Patienten neben einer humoralen auch eine zelluläre Immunantwort gegen JCV und insbesondere gegen das VP1-Protein besitzen.

### Beispiel 5:

### Verpackung von Fremdproteinen und exogener DNA in VLP des Hauptstrukturproteines VP1 von JCV

### 5.1. Verpackung von viralen Hüllglykoproteinen

Zur Verpackung eines Fremdproteins wurden in einem Modellversuch das VP1 und das Hüllglykoprotein gp160 des Affenimmundefizienzvirus SIVmac32H koexprimiert. In beiden Fällen wurde als Basisvektor für die Expression der bereits in Beispiel 1 erwähnte Vektor pBlue Bac verwendet.

Dazu wurden Sf158 Zellen in einer Multiplizität (Anzahl der infektiösen Einheiten pro Zelle) von 5 mit den für VP1 rekombinanten Baculoviren und in einer Multiplizitätsratio von gp160 zu VP1 von 0.2, 0.5 und 2 infiziert. Fünf Tage nach der Koinfektion wurden die Zellen und die Zellkulturüberstände mittels Western Blot-Analyse auf die Anwesenheit von gp160 und VP1 hin untersucht. Dabei konnte die effiziente Koexpression des gp160 und des VP1 mit einem gegen gp160 und VP1 gerichteten Antikörpergemisch nachgewiesen werden. Das gp160 und das VP1 waren ebenfalls separat nachweisbar. Dabei ergab die Multiplizitätsratio von 2 das beste Koexpressionsergebnis. Die Assoziation des gp160 an die VP1-VLP konnte nach Pelletierung des Zellkulturüberstandes durch ein 40%iges Sucrosekissen demonstriert werden. In dem resultierenden Pellet waren das gp160 separat wie auch das gp160 und das VP1 mittels des Antikörpergemisches nachweisbar.

### 5.2. Verpackung von exogener DNA

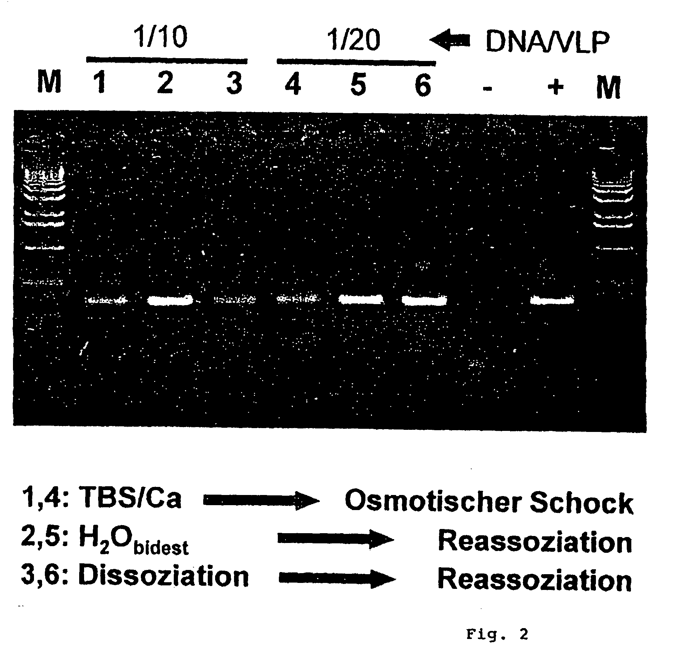

Im Rahmen der Entwicklung von effizienten Transfersystemen für die Gentherapie wurde überprüft, ob in die VP1-VLP exogene DNA verpackt werden kann. Dazu wurden drei Ansätze verfolgt. Die gereinigten VP1-VLP wurden in TBS/Ca (10 mM Tris-HCl pH: 7,5, 150 mM NaCl, 0,01 mM CaCl₂), H₂O_{bidest} oder Dissoziationspuffer (TBS pH: 8,5 mit 10 mM EGTA und 5 mM DTT) suspendiert. Exogene DNA in Form eines pUC18-Derivats, das die 1,2 kB lange V1/V2 Region des env-Gens für das Hüllglykoprotein von HIV-lhan2 trägt, dessen Sequenz bei Sauermann et al. (AIDS Research and Human Retroviruses 6 (1990), 813-823) publiziert ist (Gesamtvektor ca. 4,1 KB), wurde in einem Gewichtsverhältnis von DNA zu VP1-VLP von 1:10 und 1:20 zugegeben. Die Inkubation erfolgte für 60 Minuten bei 37°C. Der in TBS/Ca durchgeführte Ansatz wurde dann einem Osmotischen Schock unterzogen und für weitere 30 Minuten bei 37°C inkubiert. Alle Proben wurden dann über Nacht gegen Reassoziationspuffer (TBS pH: 7,5 mit 1 mM CaCl₂) dialysiert. Anschließend wurden alle Proben intensiv mit DNase I verdaut, mit TBS/Ca verdünnt und über Nacht durch ein 40%iges Sucrosekissen ultrazentrifugiert. Nach Resuspension der Partikel wurde die DNA extrahiert und die V1/V2-Region (ca. 1,8 KB) mittels spezifischer Primer über PCR amplifiziert und die Amplifikate im Agarosegel aufgetrennt. Dabei wurde gefunden, daß mit allen drei Methoden (Spuren 1 bis 6) exogene DNA in die VP1-VLP verpackt werden konnte (Fig. 2). Die exogene DNA war nach Verpackung effizient gegen den Verdau mit DNase I geschützt. Die beste Verpackungseffizienz wurde bei einem DNA zu VP1-VLP Verhältnis von 1:20 und nach Abfolge von Dissoziation und Reassoziation erreicht (Spur 6). Nach Abschätzung ausgehend von der Positivkontrolle konnten in 80 g gereinigte VP1-VLP rund 3 bis 4 g exogene DNA verpackt werden.

### Beispiel 6:

### Entwicklung eines therapeutischen Impfstoffes gegen das menschliche Polyomavirus JCV auf der Basis von VLP des Hauptstrukturproteins VP1

Zur Herstellung einer therapeutischen Vakzine gegen das menschliche Polyomavirus JCV wurde das Hauptstrukturprotein VP1 mit Hilfe rekombinanter Baculoviren in Insektenzellen exprimiert (Beispiel 1). Nach Expression bildet das VP1 typische Virus-ähnliche Partikel (VLP). Die VLP repräsentierten eine Mixtur aus "empty" und "full" Partikeln mit einem Durchmesser von rund 50 bis 60 nm. Die VP1-VLP wurden bis zur Homogenität gereinigt und zeigten eine Schwimmdichte in CsCl Gradienten von 1,32 g/ml ("empty" Partikel) und 1,34 g/ml ("full" Partikel).

### Immunogenität der VP1-VLP

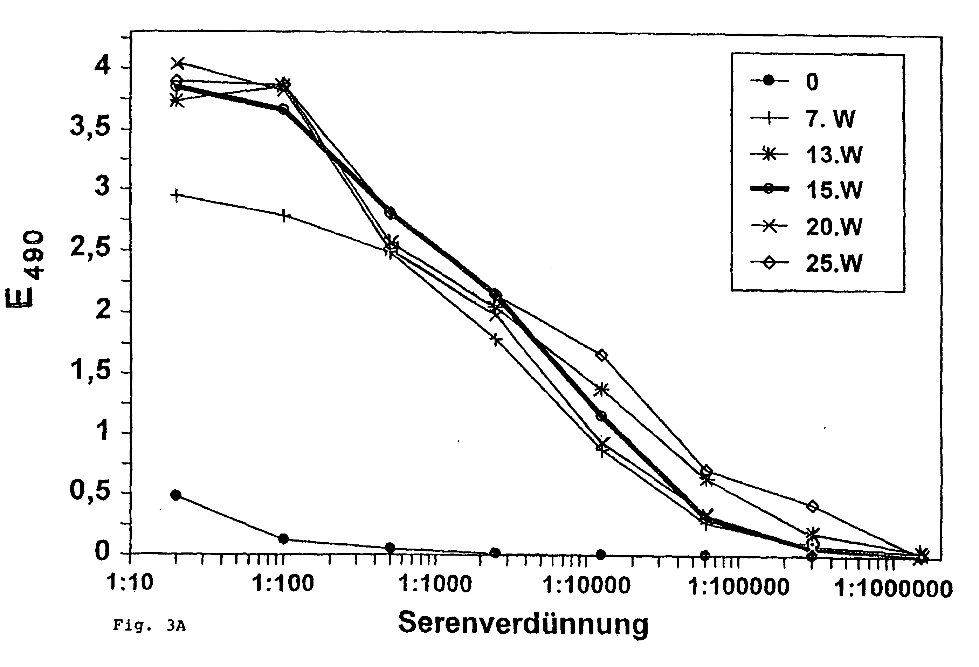

Zur Untersuchung der Immunogenität wurden 100 µg der gereinigten VP1-VLP mit 300 µg des Haemocyanin der Schlüssellochschnecke gemischt, in komplettem Freund'schen Adjuvant emulgiert und ein Kaninchen intramuskulär immunisiert. Eine Auffrischungimmunisierung erfolgte vier Wochen später mit inkomplettem Freund'schem Adjuvant. In Figur 3A ist die Analyse des Titers von Anti-VP1 Antiserum im ELISA von verschiedenen Abnahmedaten nach der letzten Immunisierung des Kaninchens dargestellt. Für die Untersuchungen wurden 50 ng der gereinigten VP1-VLP an eine ELISA-Platte (Greiner, Nürtingen) absorbiert und das Anti-VP1 Antiserum der entsprechenden Abnahmedaten (+: 7 Wochen; *: 13 Wochen, □: 15 Wochen; X: 20 Wochen; ◇: 25 Wochen) in den angegebenen Verdünnungen austitriert. Der Endpunkttiter wurde als die Verdünnung definiert, bei der die Reaktivität der des Präimunserums (-) entsprach. Wie in Figur 3A zu sehen ist, hatte das Immunserum sechs Wochen nach der letzten Immunisierung einen Endpunkttiter von rund 10⁵ erreicht.

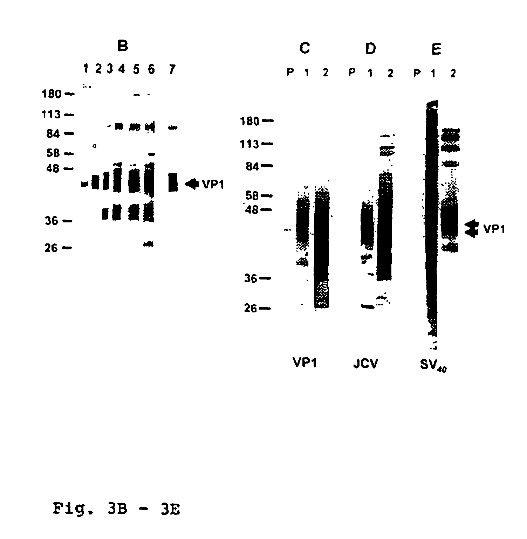

Die Spezifität des Anti-VP1 Antiserums wurde im Western-Blot (WB) untersucht. Hierfür wurden rd. 20 ng gereinigtes Antigen pro Spur mittels SDS-Polyacrylamidgelelektrophorese aufgetrennt und auf eine Nitrozellulosemembran übertragen. In Figur 3B wurden als Antigen die VPS1-VLP verwendet und die Reaktivität des anti-VP1 Antiserums verschiedener Abnahmedaten (Spur 2: 7 Wochen; Spur 3: 13 Wochen; Spur 4: 15 Wochen; Spur 5: 20 Wochen; Spur 6: 25 Wochen) mit der Reaktivität eines Präimmunserums (Spur 1) und eines anti-SV40 Hyperimmunserums (Spur 7) verglichen. In Figur 3C wurden ebenfalls gereinigte VP1-VLP verwendet, während in Figur 3B gereinigte natürliche JCV Partikel und in Firgur 3E gereinigte natürliche SV40 Partikel als Antigen finden. In den Figuren C-E repräsentierten die jeweilige Spur P das Prämimmunserum, die jeweilige Spur 1 das antiSV40 Immunserum und die jeweilige Spur 2 das anti-VPS Antiserum.

Die Spezifität des Antiserums entsprach den Erwartungen. Wie eine Western Blot-Analyse zeigte, wurden lediglich VP1-VLP-spezifische Proteine erkannt und ein deutlicher Anstieg der Reaktivität in Abhängigkeit von dem Blutungszeitpunkt nach der letzten Immunisierung konnte beobachtet werden (Fig. 3B, Spuren 2 bis 6). Die Reaktivität war vergleichbar einem anti-SV40 Hyperimmunserum (Spur 7). Die Immunreaktivität des Anti-VP1-VLP Immunserums wurde ebenfalls gegen verschiedene Antigene getestet. Das Hyperimmunserum erkannte zusätzlich zu dem rekombinanten VP1 (Figur 3C, Spur 1) das VP1 von SV40 (Fig. 3D, Spur 1) und das VP1 von natürlichem JCV (Fig.3E, Spur 1). Wiederum war die Reaktivität der des anti-SV40 Immunserums vergleichbar (Figuren 3C bis E, jeweilige Spuren 2).

### Bindung und Bindungsinhibition der VP1-VLP an SVG Zellen

Für diese Versuche wurden die VP1-VLP mit ¹²⁵J radioaktiv markiert. Zunächst wurde die Ratio der ¹²⁵J-VP1-VLP und SVG Zellen bestimmt, die durch Transfektion humaner fötaler Gliazellen mit einer im Replikationsursprung defekten SV 40-Mutante erhalten wurden (Major et al., Proc. Natl. Acad. Sei USA 82 (1985), 1257-1261). Mit 2x10⁶ cpm ¹²⁵J-VP1-VLP und 10⁵ SVG Zellen wurde eine Sättigungsbindung erreicht. Unter diesen Testbedingungen wurde die Bindungsinhibition des anti-VP1-VLP Immunserums untersucht. Bei Serumverdünnung von 1:5 und 1:10 konnte die ¹²⁵J-VP1-VLP Bindung zu 95% unterdrückt werden und bei einer Serumverdünnung von 1:20 war immer noch eine Bindungsinhibition von rund 75% zu beobachten. Während bei einer Serumverdünnung von 1:40 nur noch ein 20%ige Bindungsinhibition meßbar war, wurde bei einer Serumverdünnung von 1:80 keine Bindungsinhibition erreicht.

### Neutralisation von JCV

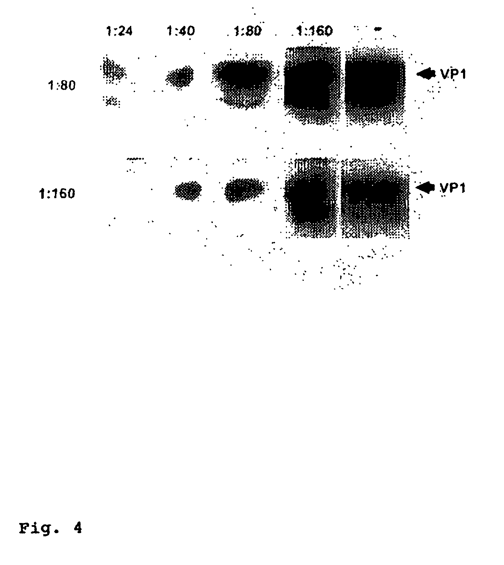

Zur Untersuchung der Neutralisationskapazität des VP1-VLP Hyperimmunserums wurde ein neuer Test entwickelt. Dieser Test beruht auf dem intrazellulären Nachweis von VP1 nach erfolgter Infektion von SVG Zellen mit JCV. Wie aus Figur 4 hervorgeht, konnte bei einer Verdünnung des JCV-Infektionsstocks von 1:160 und einer Serumverdünnung von 1:24 eine vollständige Neutralisation von JCV erreicht werden, da kein VP1 nachweisbar war. Bei einer Serumverdünnung von 1:40 wurde wohl VP1 erkannt, aber in einer reduzierten Form, was auf eine partielle JCV-Neutralisation hinweist. Dies traf auch auf eine Serumverdünnung von 1:80 zu, während bei einer Serumverdünnung von 1:160 im Vergleich zur Kontrolle keine Neutralisationseffekte erzielt wurden. Ähnliche Ergebnisse wurden auch bei einer Verdünnung des JCV-Infektionsstocks von 1:80 erzielt.

Diese Ergebnisse zeigen, daß die VP1-VLP eine Immunantwort induzieren, wie sie von einem Impfstoff mit Erfolgspotenial zu erwarten sind. Es werden neutralisierende und bindungsinhibierende Antikörper induziert. Darüberhinaus sind die VP1-VLP dazu in der Lage, eine proliferative T-Zellreaktivität in den peripheren Blutlymphozyten von JCV-positiven, aber gesunden Individuen zu induzieren (Beispiele 2-4).

## Patentansprüche

1. Virus-ähnliches Partikel (VLP), das aus mehreren Molekülen des Virusprotein 1 (VP1) von JC-Virus aufgebaut ist,
**dadurch gekennzeichnet,**
**daß** das VP1 codiert ist von einer Nukleinsäure, welche
(a) die in SEQ ID NO. 1 gezeigte Nukleotidsequenz,
(b) eine der Sequenz aus (a) im Rahmen der Degeneration des genetischen Codes entsprechende Nukleotidsequenz oder/und
(c) eine mit einer der Sequenzen aus (a) oder/und (b) unter stringenten Bedingungen, umfassend einen Waschschritt von 30 min in 0,1 x SSC, 0,5 % SDS bei 68 °C, hybridisierende Nukleotidsequenz umfaßt.

2. Partikel nach Anspruch 1,
**dadurch gekennzeichnet,**
**daß** es aus rekombinantem VP1 aufgebaut ist.

3. Partikel nach Anspruch 1 - 2,
**dadurch gekennzeichnet,**
**daß** es weiterhin mindestens ein zusätzliches heterologes Protein in der Kapsidstruktur inkorporiert aufweist.

4. Partikel nach Anspruch 3,
**dadurch gekennzeichnet,**
**daß** das zusätzliche Protein ein Zelloberflächenrezeptor oder ein Bindungspartner für einen Zelloberflächenrezeptor ist.

5. Partikel nach Anspruch 4,
**dadurch gekennzeichnet,**
**daß** das zusätzliche Protein ein virales Oberflächenprotein ist.

6. Partikel nach Anspruch 4 oder 5,
**dadurch gekennzeichnet,**
**daß** das zusätzliche Protein gp120 von HIV ist.

7. Partikel nach einem der Ansprüche 1 - 6,
**dadurch gekennzeichnet,**
**daß** es im Inneren der Kapsidstruktur mindestens eine Wirksubstanz enthält.

8. Partikel nach Anspruch 7,
**dadurch gekennzeichnet,**
**daß** die Wirksubstanz ausgewählt ist aus Nukleinsäuren, Proteinen und physiologisch aktiven Stoffen.

9. Partikel nach Anspruch 8,
**dadurch gekennzeichnet,**
**daß** die Wirksubstanz eine Nukleinsäure umfaßt.

10. Verfahren zur Herstellung von VLP nach einem der Ansprüche 1 - 9,
**dadurch gekennzeichnet,**
**daß** man eine für ein VP1-Protein codierende Nukleinsäure in eine Zelle einbringt, die transformierte Zelle in einem Medium unter Bedingungen kultiviert, bei denen eine Expression der Nukleinsäure erfolgt, das VP1-Protein aus der Zelle oder aus dem Medium gewinnt, aufreinigt und zum VLP assembliert.

11. Verfahren nach Anspruch 10,
**dadurch gekennzeichnet,**
**daß** die für ein VP1 Protein codierende Nukleinsäure
(a) die in SEQ ID NO. 1 gezeigte Nukleotidsequenz,
(b) eine der Sequenz aus (a) im Rahmen der Degeneration des genetischen Codes entsprechende Nukleotidsequenz oder/und
(c) eine mit einer der Sequenzen aus (a) oder/und (b) unter stringenten Bedingungen, umfassend einen Waschschritt von 30 min in 0,1 x SSC, 0,5 % SDS bei 68 °C, hybridisierende Nukleotidsequenz umfaßt.

12. Verfahren nach einem der Ansprüche 10 oder 11,
**dadurch gekennzeichnet,**
**daß** die für ein VP1 Protein codierende Nukleinsäure in mindestens einer Kopie auf einem rekombinanten Vektor, insbesondere unter Kontrolle eines Expressionssignals, lokalisiert ist.

13. Verfahren nach einem der Ansprüche 10 bis 12,
**dadurch gekennzeichnet,**
**daß** man eine Insektenzelle verwendet.

14. Verfahren nach einem der Ansprüche 10 - 13,
**dadurch gekennzeichnet,**
**daß** man die Assemblierung in Gegenwart mindestens eines weiteren, heterologenen Proteins durchführt, wobei das Protein in die Kapsidhülle inkorporiert wird.

15. Verfahren nach einem der Ansprüche 10 - 14,
**dadurch gekennzeichnet,**
**daß** man die Assemblierung in Gegenwart einer weiteren Substanz durchführt, wobei die Substanz im Inneren der Kapsidhülle eingeschlossen wird.

16. Verfahren nach Anspruch 15,
**dadurch gekennzeichnet,**
**daß** man die Assemblierung in Gegenwart einer Nukleinsäure durchführt.

17. Verfahren zur immunologischen Bestimmung spezifischer Antikörper gegen JCV in einer Probe,
**dadurch gekennzeichnet,**
**daß** man die Antikörper durch Bindung an ein VLP nach einem der Ansprüche 1 - 9 oder einem Bestandteil davon qualitativ oder/und quantitativ nachweist.

18. Verfahren nach Anspruch 17,
**dadurch gekennzeichnet,**
**daß** man ein aus rekombinanten VP1-Molekülen aufgebautes VLP verwendet.

19. Verfahren nach Anspruch 17 oder 18,
**dadurch gekennzeichnet,**
**daß** man die VLP oder Bestandteile davon mit der Probe unter geeigneten Bedingungen in Kontakt bringt, um die Bindung spezifischer Antikörper gegen JC-Virus an die VLP bzw. deren Bestandteile zu gestatten, die gebildeten Immunkomplexe von anderen Probenbestandteilen abtrennt und die Gegenwart von Antikörpern nachweist.

20. Verfahren nach einem der Ansprüche 17 - 19 zur Diagnose für progressive multifokale Leukoenzephalopathie (PML),
**dadurch gekennzeichnet,**
**daß** man als Proben Cerebrospinalflüssigkeit und Serum, die aus dem gleichen Patienten stammen, verwendet.

21. Testkit zur Bestimmung spezifischer Antikörper gegen JC-Virus,
**dadurch gekennzeichnet,**
**daß** er in getrennter räumlicher Anordnung VLP nach einem der Ansprüche 1 - 9 oder Bestandteile davon, Mittel zum Nachweis von Antikörpern und gegebenenfalls übliche Puffer und Hilfssubstanzen umfaßt.

22. Testkit nach Anspruch 21,
**dadurch gekennzeichnet,**
**daß** das Mittel zum Nachweis von Antikörpern einen spezifischen mit den nachzuweisenden Antikörpern bindefähigen Rezeptor und geeignete Detektionsmittel umfaßt.

23. Verwendung eines VLP nach einem der Ansprüche 1 - 9 zur Herstellung eines Mittels zur Therapie von PML.

24. Verwendung eines VLP nach einem der Ansprüche 1 - 9 zur Herstellung eines therapeutischen oder/und prophylaktischen Impfstoffes gegen eine JVC-Infektion.

25. Verwendung eines VLP nach Anspruch 7 oder 8 als Transportvehikel.

26. Verwendung nach Anspruch 25 zur Herstellung eines Transportersystems für die Gentherapie.

27. Verwendung nach Anspruch 25,
**dadurch gekennzeichnet,**
**daß** die in der Kapsidhülle eingeschlossene Substanz eine Antisense-Nukleinsäure ist.

28. Verwendung nach Anspruch 27,
**dadurch gekennzeichnet,**
**daß** die Antisense-Nukleinsäure eine TNF-α-Antisense-Nukleinsäure ist.

29. Pharmazeutische Zusammensetzung, umfassend ein VLP nach einem der Ansprüche 1 - 9 sowie gegebenenfalls übliche Puffer, Hilfs-, Zusatz- oder/und Verdünnungsmittel.

## Claims

1. Virus-like particle (VLP) which is composed of several molecules of the virus protein (VP1) of the JC virus,
**characterized in that**
the VP1 is coded by a nucleic acid which comprises
(a) the nucleotide sequence shown in SEQ ID NO.1
(b) a nucleotide sequence corresponding to the sequence from (a) within the scope of the degeneracy of the genetic code or/and
(c) a nucleotide sequence hybridizing with one of the sequences from (a) or/and (b) under stringent conditions that comprise a wash step of 30 min in 0.1 x SSC, 0.5 % SDS at 68°C.

2. Particle as claimed in claim 1,
**characterized in that**
it is composed of recombinant VP1.

3. Particle as claimed in claims 1 - 2,
**characterized in that**
it additionally has at least one additional heterologous protein incorporated into the capsid structure.

4. Particle as claimed in claim 3,
**characterized in that**
the additional protein is a cell surface receptor or a binding partner for a cell surface receptor.

5. Particle as claimed in claim 4,
**characterized in that**
the additional protein is a viral surface protein.

6. Particle as claimed in claim 4 or 5,
**characterized in that**
the additional protein is gp120 from the HIV.

7. Particle as claimed in one of the claims 1 - 6,
**characterized in that**
it contains at least one active substance inside the capsid structure.

8. Particle as claimed in claim 7,
**characterized in that**
the active substance is selected from nucleic acids, proteins and physiologically active substances.

9. Particle as claimed in claim 8,
**characterized in that**
the active substance comprises a nucleic acid.

10. Process as claimed in one of the claims 1 - 9,
**characterized in that**
a nucleic acid coding for a VP1 protein is introduced into a cell, the transformed cell is cultured in a medium under conditions in which the nucleic acid is expressed, the VP1 protein is isolated from the cell or from the medium, purified and assembled to form VLP.

11. Process as claimed in claim 10,
**characterized in that**
the nucleic acid coding for a VP1 protein comprises
(a) the nucleotide sequence shown in SEQ ID NO.1
(b) a nucleotide sequence corresponding to the sequence from (a) within the scope of the degeneracy of the genetic code or/and
(c) a nucleotide sequence hybridizing with one of the sequences from (a) or/and (b) under stringent conditions that comprise a wash step of 30 min in 0.1 x SSC, 0.5 % SDS at 68°C.

12. Process as claimed in one of the claims 10 or 11,
**characterized in that**
the nucleic acid coding for a VP1 protein is present in at least one copy of a recombinant vector in particular under the control of an expression signal.

13. Process as claimed in one of the claims 10 to 12,
**characterized in that**
an insect cell is used.

14. Process as claimed in one of the claims 10 - 13,
**characterized in that**
the assembly is carried out in the presence of at least one additional heterologous protein during which the protein is incorporated into the capsid envelope.

15. Process as claimed in one of the claims 10 - 14,
**characterized in that**
the assembly is carried out in the presence of a further substance, said substance being enclosed in the interior of the capsid envelope.

16. Process as claimed in claim 15,
**characterized in that**
the assembly is carried out in the presence of a nucleic acid.

17. Method for the immunological determination of specific antibodies against JCV in a sample,
**characterized in that**
the antibodies are detected qualitatively or/and quantitatively by binding to a VLP as claimed in one of the claims 1 - 9 or to a component thereof.

18. Method as claimed in claim 17,
**characterized in that**
a VLP is used which is composed of recombinant VP1 molecules.

19. Method as claimed in claim 17 or 18,
**characterized in that**
the VLP or components thereof are contacted with the sample under suitable conditions in order to allow binding of specific antibodies against JC virus to the VLP or to components thereof, the formed immune complexes are separated from other sample components and the presence of antibodies is detected.

20. Method as claimed in one of the claims 17 - 19 for the diagnosis of progressive multifocal leukoencephalopathy (PML),
**characterized in that**
cerebrospinal fluid and serum are used as the samples which are derived from the same patient.

21. Test kit for the determination of specific antibodies against a JC virus,
**characterized in that**
it comprises in a separate spatial arrangement VLP as claimed in one of the claims 1 - 9 or components thereof, an agent for the detection of antibodies and optionally common buffers and auxiliary substances.

22. Test kit as claimed in claim 21,
**characterized in that**
the agent for the detection of antibodies comprises a specific receptor that is capable of binding to the antibodies to be detected and suitable detection agents.

23. Use of a VLP as claimed in one of the claims 1 - 9 to produce an agent for the treatment of PML.

24. Use of a VLP as claimed in one of the claims 1 - 9 for the production of a therapeutic or/and prophylactic vaccine against JVC infection.

25. Use of a VLP as claimed in claim 7 or 8 as a transport vehicle.

26. Use as claimed in claim 25 to produce a transporter system for gene therapy.

27. Use as claimed in claim 25,
**characterized in that**
the substance enclosed in the capsid envelope is an antisense nucleic acid.

28. Use as claimed in claim 27,
**characterized in that**
the antisense nucleic acid is a TNF-α antisense nucleic acid.

29. Pharmaceutical composition comprising a VLP as claimed in one of the claims 1 - 9 as well as optionally common buffers, auxiliary substances, additives or/and diluents.

## Revendications

1. Particule pseudovirale (VLP) assemblée à partir de plusieurs molécules de la protéine virale 1 (VP1) du virus JC,
**caractérisée en ce que**
la VP1 est codée par un acide nucléique qui comprend
(a) la séquence de nucléotides indiquée dans SEQ ID NO. 1
(b) une séquence de nucléotides correspondant à la séquence de (a) dans le cadre de la dégénérescence du code génétique ou/et
(c) une séquence de nucléotides s'hybridant avec l'une des séquences de (a) et/ou de (b) dans des conditions stringentes, comprenant une étape de lavage de 30 min dans 0,1 x SSC, 0,5 % de SDS à 68°C.

2. Particule selon la revendication 1
**caractérisée en ce**
**qu'**elle est assemblée à partir de VP1 recombinante.

3. Particule selon la revendication 1-2
**caractérisée en ce que**
elle présente en outre au moins une protéine hétérologue supplémentaire incorporée dans la structure de capside.

4. Particule selon la revendication 3,
**caractérisée en ce que**
la protéine supplémentaire est un récepteur de surface cellulaire ou un ligand pour un récepteur de surface cellulaire.

5. Particule selon la revendication 4,
**caractérisée en ce que**
la protéine supplémentaire est une protéine de surface virale.

6. Particule selon la revendication 4 ou 5,
**caractérisée en ce que**
la protéine supplémentaire est gp120 de HIV.

7. Particule selon l'une quelconque des revendications 1 à 6,
**caractérisée en ce que**
à l'intérieur de la structure de capside, elle contient au moins une substance active.

8. Particule selon la revendication 7,
**caractérisée en ce que**
la substance active est choisie parmi les acides nucléiques, les protéines et les substances physiologiquement actives.

9. Particule selon la revendication 8,
**caractérisée en ce que**
la substance active comprend un acide nucléique.

10. Procédé de préparation de VLP selon l'une quelconque des revendications 1 à 9,
**caractérisé en ce que**
on incorpore un acide nucléique codant une protéine VP1 dans une cellule, on cultive la cellule transformée dans un milieu dans des conditions propices à une expression de l'acide nucléique, on obtient la protéine VP1 à partir de la cellule ou du milieu, on la purifie et on l'assemble VLP.

11. Procédé selon la revendication 10,
**caractérisé en ce que**
l'acide nucléique codant pour une protéine VP1 comprend
(a) la séquence de nucléotides indiquée dans SEQ ID NO. 1
(b) une séquence de nucléotides correspondant à la séquence de (a) dans le cadre de la dégénérescence du code génétique ou/et
(c) une séquence de nucléotides s'hybridant avec l'une des séquences de (a) et/ou de (b) dans des conditions stringentes, comprenant une étape de lavage de 30 min dans 0,1 x SSC, 0,5 % de SDS à 68°C.

12. Procédé selon l'une quelconque des revendications 10 ou 11,
**caractérisé en ce que**
l'acide nucléique codant pour une protéine VP1 est localisé, en au moins une copie, sur un vecteur recombinant, en particulier sous le contrôle d'un signal d'expression.

13. Procédé selon l'une quelconque des revendications 10 à 12,
**caractérisé en ce que**
on utilise une cellule d'insecte.

14. Procédé selon l'une quelconque des revendications 10 à 13,
**caractérisé en ce que**
on procède à l'assemblage en présence d'au moins une autre protéine hétérologue, la protéine étant incorporée dans l'enveloppe de la capside.

15. Procédé selon l'une quelconque des revendications 10 à 14,
**caractérisé en ce que**
on procède à l'assemblage en présence d'une autre substance, ladite substance étant incluse à l'intérieur de l'enveloppe de la capside.

16. Procédé selon la revendication 15,
**caractérisé en ce que**
on procède à l'assemblage en présence d'un acide nucléique.

17. Procédé de détermination immunologique d'anticorps spécifiques contre le JCV dans un échantillon,
**caractérisé en ce que**
on détecte qualitativement et/ou quantitativement les anticorps par liaison à une VLP selon l'une quelconque des revendications 1-9 ou un constituant de celle-ci.

18. Procédé selon la revendication 17,
**caractérisé en ce que**
on utilise une VLP assemblée à partir de molécules VP1 recombinantes.

19. Procédé selon la revendication 17 ou 18
**caractérisé en ce que**
on met en contact la VLP ou des constituants de celle-ci avec l'échantillon dans des conditions adaptées pour permettre la liaison d'anticorps spécifiques contre le virus JC à la VLP respectivement à ses constituants, on sépare les complexes immuns formés d'autres constituants de l'échantillon et on détecte la présence d'anticorps.

20. Procédé selon l'une quelconque des revendications 17 à 19 destiné au diagnostic de la leucoencéphalopathie multifocale progressive (PML),
**caractérisé en ce que**
on utilise comme échantillons du liquide cérébro-spinal et du sérum qui proviennent du même patient.

21. Kit de test pour la détermination d'anticorps spécifiques contre le virus JC,
**caractérisé en ce que**
il comprend, dans une disposition séparée dans l'espace, une VLP selon l'une quelconque des revendications 1 à 9 ou des constituants de celle-ci, des moyens de détection d'anticorps et, le cas échéant, des tampons et substances auxiliaires usuels.

22. Kit de test selon la revendication 21,
**caractérisé en ce que**
le moyen de détection d'anticorps comprend un récepteur spécifique capable de se lier avec les anticorps à détecter et des agents de détection appropriés.

23. Utilisation d'une VLP selon l'une quelconque des revendications 1 à 9 pour la préparation d'un moyen destiné à traiter la PML.

24. Utilisation d'une VLP selon l'une quelconque des revendications 1 à 9 pour la préparation d'un vaccin thérapeutique et/ou prophylactique contre une infection par le JVC.

25. Utilisation d'une VLP selon la revendication 7 ou 8 en tant que vecteur de transport.

26. Utilisation selon la revendication 25 pour la préparation d'un système de transport destiné à la thérapie génique.

27. Utilisation selon la revendication 25,
**caractérisée en ce que**
la substance incluse dans l'enveloppe de la capside est un acide nucléique antisens.

28. Utilisation selon la revendication 27,
**caractérisée en ce que**
l'acide nucléique antisens est un acide nucléique antisens de TNF-α.

29. Composition pharmaceutique comprenant une VLP selon l'une quelconque des revendications 1 à 9 ainsi que, le cas échéant, des tampons, des agents auxiliaires, des additifs et/ou des diluants usuels.
